# EUROPEAN PATENT SPECIFICATION

(11) **EP 0 767 649 B1**
(45) Date of publication and mention of the grant of the patent: **11.08.1999**
(21) Application number: 95925757.7
(22) Date of filing: 24.06.1995
(51) Int. Cl.: A61F 13/15

(54) **CORRUGATED TOP LAYER FOR ABSORBENT ARTICLES**
WELLENFORMIGE OBERSCHICHT FÜR ABSORBIERNDE ARTIKEL
COUCHE SUPERIEURE ONDULEE DESTINEE A DES ARTICLES ABSORBANTS

(30) Priority: 30.06.1994 DE 4422956
(43) Date of publication of application: 16.04.1997
(73) Proprietor: THE PROCTER & GAMBLE COMPANY, Cincinnati, Ohio 45202 (US)
(72) Inventor: RAIDEL, Maria, D-90451 Nürnberg (DE)
(74) Representative: Hübner, Gerd, Dipl.-Phys.
(86) International application number: PCT/EP95/02466
(87) International publication number: WO 96/00545

(56) References cited:
- EP-A- 0 321 985
- EP-A- 0 529 641
- WO-A-91/11161
- WO-A-93/19711
- GB-A- 2 023 067

## Description

The invention relates to an absorbent sanitary product for the absorption of body fluid, such as sanitary napkins, diapers, incontinence articles or the like, with the features mentioned in the preamble of claim 1. Such a product is disclosed in GB-A-2023 067 for example.

Sanitary products of this type are well-known in a large variety. They are all provided with a liquid-proof covering layer on the back and a liquid-permeable covering layer on the top with an absorbent body inserted between these two layers.

The covering layer on the back usually consists of a thin polyethylene foil. The liquid-permeable covering layer is mostly of nonwoven material, but perforated foils can also be used.

Normally the absorbent body is produced of cellulosic flocs or so-called air-laid-material.

In addition ot the basic absorptive capacity of the absorbent body the product's technical features of the body-facing covering layer such as the tactile softness, absorbing time in relation to the body liquids, the distribution over the sanitary product and the rewet properties are of vital importance. The nonwoven covers normally used for the body-facing cover layer are comparable in most of the above-mentioned features.

The previously mentioned perforated foils have been improved in their absorptive capacity and in the visual impression after admission of menstruation liquid for instance, whereas impairments might occur by the plastic-like character of the perforated foils which causes perspiration.

Above-mentioned properties of the body-facing covering layer regarding the absorbing time and distribution of incurring body liquids and rewetting have a major impact on the leaking safety of the respective sanitary product Due to the technical standard a variety of measures are known which influence these factors positively. For instance a transport layer has been inserted under the body-facing covering layer to improve the absorbing time and the rewet properties. Improvements on the liquid distribution in the longitudinal direction of mentioned sanitary products - which lead to an increased safety against lateral leakage of the body liquid - have been substantially obained by impressed, longitudinal lines on the surface of a sanitary napkin.

A substantial contribution regarding the sanitary product's leaking safety are so-called cuffs, which, arranged on both of the lateral longitudinal margins of the absorbent body, form a liquid barrier that stays upright by means of elastified material and hinders lateral leakage of the body liquid. While these cuffs greatly increase the leaking safety, they considerably complicated the production of sanitary napkings, diapers, incontinence articles and the like.

From the cited reference GB-A-20 23 067 it is also known to provide the liquid-permeable top sheet with a plurality of folds following the longitudinal direction of the product. These folds are evenly distributed over the width of the sanitary product. The foot areas of the folds are attached to the absorbent body of the sanitary product.

It is an object of the invention to modify sanitary products of the generic type in a manner that the arrangement of folds is optimized concerning the distribution and absorption of body liquids on the one hand and low manufacturing efforts concerning the pleating of a top sheet on the other hand.

This object is obtained by the characterizing features of claim 1, according to which the folds are arranged in stripes on the absorbent body's surface, which run along the margins and/or in the center.

Furthermore it is generally to be noted that the folds in most areas of the covering layer built a spacing between themselves and the absorbent body underneath, which improves both the absorptive or penetrating action of the body liquid into the absorbent body and the rewet properties. The visual impression after admission of for instance menstruation liquid will also be improved because raised folded areas are not in contact with the liquid-soaked absorbent body. The flexibility of the folds will also have an effect on the softness of the body-facing surface of the sanitary product thus providing a higher wearing comfort and the natural comfort and the natural touch.

Due to the fixing of folds these are stabilized in such a manner, that the created positive effects will remain, even after storage in pressed condition and over a extended wearing period.

Besides according to the preamble of claim 1 the foot area of the folds can be directly fastened by glueing or welding on the absorbent body or on a support layer which is inserted between the covering layer and the absorbent body. This support layer helps to improve the functional features of the folds regarding the further transmission of liquid and the rewet properties.

Claims 2 and 3 indicate advantageous dimensional ranges for the folds.

Additional features, details and advantages of the invention can be taken from the following description, in which various embodiments of the invention are explained referring to the attached drawings.
- Fig. 1: is a perspective partial view of a sanitary napkin, known from prior art
- Fig. 2: is a cross-section of a sanitary napkin according to Fig. 1 with a partial enlargement,
- Fig. 3: is a cross-section of a further known sanitary napkin,
- Fig. 4: is a cross-section of a sanitary napkin according to the invention, and
- Fig. 5: is a schematic perspective view of a sanitary napkin's surface in a further embodiment of the invention.

As can be seen in the attached drawings, the different types of the respective sanitary napkin all have a base structure which is identical in vital parts. The back is provided with a liquid-proof covering layer, the underwear protecting foil 1, which is underneath the absorbent body 2 consisting of a pad of cellulosic flocs in the area of its back 3 - i.e. on the body-averting side of the sanitary napkin when worn. Furthermore the longitudinal edges 4 of the underwear protecting foil 1 reach over the lateral margin 5 of the absorbent body.

A support layer 7 for instance made of nonwoven material is arranged on the front surface area 6. The longitudinal edges 8 of this support layer 7 also reach over the lateral margin 5 of the absorbent body 2 and overlap the longitudinal edges 4 of the underwear protecting foil 1. Both of these parts are glued together in the usual manner in the overlapping area.

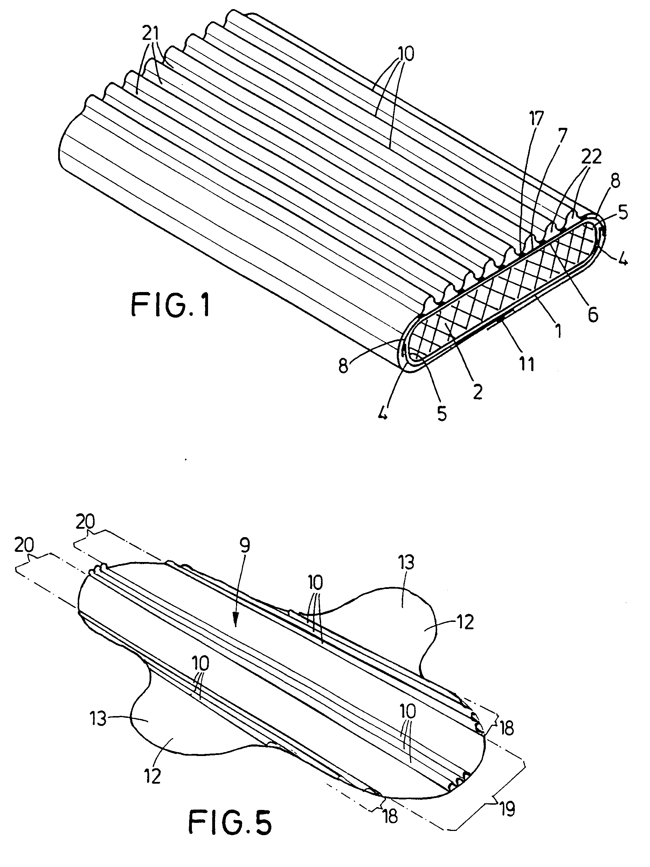

On top of support layer 7 a liquid-permeable covering layer 9 is arranged which is provided with folds 10 as later described in detail. The embodiments of the sanitary napkin shown in Fig. 1, 2 and 4 are a so-called "fully wrapped sanitary napkin", which consists of a covering layer 9 - for instance of polypropylene card web - and encloses the absorbent body 2 and the support layer 7 . Covering layer 9 is folded on the back 3 and attached to the center by means of a glued connection 11.

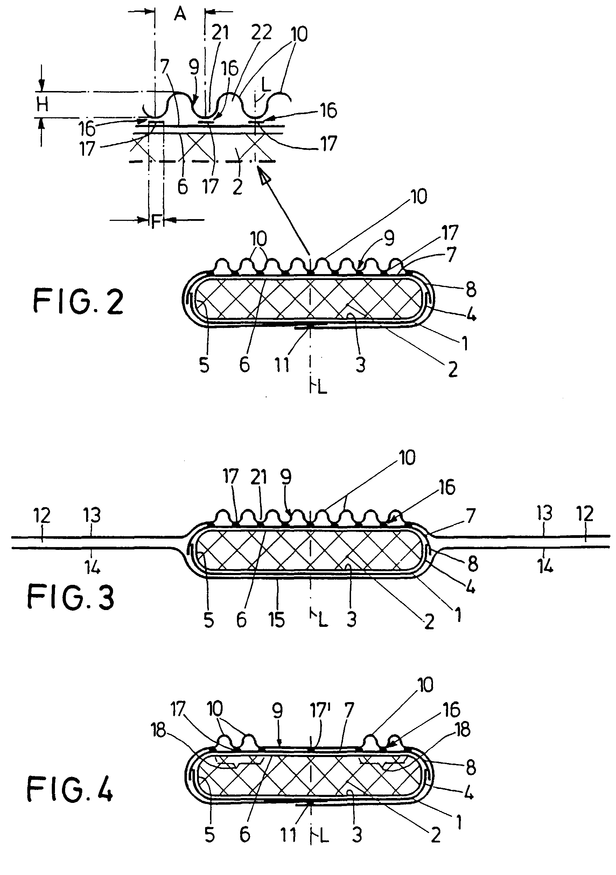

The embodiments according to Fig. 3 and 5 are provided with lateral wings 12, which are formed by overlapping flaps 13, 14 being connected to the lateral margins of covering layer 9 on the front and covering layer 15 on the back. The covering layer 15 might be of nonwoven material such as the case in the embodiment shown in Fig. 3, which shall be provided with a separate underwear protecting foil 1. The covering layer can also consist of liquid-proof material, for instance of polyethylene foil, thus taking over the underwear protecting foil's function and omitting it.

Now the different configurations of folds used in illustrated sanitary napkins shall be explained in more detail.

The folds 10 are formed by a suitable folding device in the sanitary napkin's production machine so that the foot areas 16 are located on support layer 7 with a spacing of 1 to 20 mm in the transversal direction of the sanitary napkin. A spacing of 3 to 5 mm has proven to be most practicable. The foot areas 16 are attached to the support layer 7 by means of longitudinal glue strips 17, for instance of spray adhesive. Between each adjacent foot area 16 the fold 10 stands upright with a height H ranging from 1 to 20 mm. The most practicable values for the height were also in the area of 3 to 5 mm. The foot width F defined by the glue strips 17 can vary from 0.2 to 10 mm depending on the spacing A and the height H, whereas it must be considered that the maximum foot width F is half the spacing A. With spacing A and height H in the range of 3 to 5 mm, foot widths of 0.5 to 1 mm proved to be successful.

In the embodiments shown in Fig. 1 through 3 eight folds are provided evenly spread over the entire width of the absorbent body's surface, working with a spacing A of approx. 6 mm between the folds.

In the embodiment shown in Fig 4 within two lateral strips 18 two folds 10, respectively, are provided, which run in the longitudinal direction of the sanitary napkin and which have a spacing A and height H of approx. 6 mm. The remaining center strip 19 between both lateral strips 18 consists of a liquid-permeable covering 9 which lays evenly on top of the support layer 7 to which it is additionally fastened by a center glue strip 17'. A liquid-permeable glueing over the entire area can also be obtained with amall quantity of adhesive.

The embodiment of a sanitary napkin shown in Fig. 5 has in addition to folds 10 on both lateral strips 18 further folding configurations, symmetrical to center plane L consisting of three folds 10, whereas a strip without folds 20 remains between the center folds 10 and the lateral folds 10.

Like noticeable from the cross-sections according to Fig. 2 through 4, the top of the folds 10, the chamfers 21 in between and the tubes 22 which are underneath the folds 10 between the glued foot areas 16 built channels for the longitudinal distribution of body liquids admitted on the sanitary napkin.

Closing it has to be pointed out that the individual components of the invented sanitary napkin shall be of suitable material available on the market according to common criteria for the production of these sanitary products. Suitable constructional measures shall be utilized. For instance it is advisable to glue the strips 17 on the support layer (7) surface in such manner, that they hinder the penetration of liquids only to a very small extent that the absorbent surface of the sanitary napkin still remains relatively high. Spray adhesive or small threads of melt-blown adhesive can be used, another possibility is welding or sealing by laser, ultrasound or heat.

## Claims

1. An absorbent sanitary product for the absorption of body liquids, especially sanitary napkins, diapers, incontinence napkins or the like comprising
- a liquid-proof covering layer (1) on the back,
- a liquid-permeable covering layer (9), preferrably of nonwoven material, on the top and
- a absorbent body (2) inserted between the back and top covering layer (9, 1),
wherein the top covering layer (9) in the area of the absorbent body (2) is arranged in two or more folds (10) following the longitudinal direction of the product, wherein the foot areas (16), directing towards the absorbent body (2) of the folds (10), are attached to the absorbent body (2) or to a support layer (7) inserted between the absorbent body (2) and the top covering layer (9) and wherein the foot areas (16) of the folds (10) are glued (17) or welded on the absorbent body (2) or the support layer (7) over a width (F) of preferrably 0.2 - 10 mm, characterized in that several, preferrably 2 - 10 folds (10) are provided on a partial lateral strip (18) of the absorbent body's surface (6) and/or a center strip (19).

2. A sanitary product according to claim 1, characterized in that the foot areas (13) of two adjacent folds (10) are fixed with a mutual spacing (A) of 1 - 20 mm.

3. A sanitary product according to claim 1 or 2, characterized in that the height (H) of the folds (10) is 1 - 20 mm in no-load condition.

## Patentansprüche

1. Ein absorbierendes Hygieneprodukt zur Absorption von Körperflüssigkeiten, insbesondere Hygienevorlagen, Windeln, Inkontinenzvorlagen oder dergleichen, welches umfaßt
- eine flüssigkeitsbeständige Deckschichte (1) an der Unterseite,
- eine flüssigkeitsdurchlässige Deckschichte (9), vorzugsweise ein Faservliesmaterial, an der Oberseite und
- einen absorbierenden Körper (2), welcher zwischen die untere und die obere Deckschichte (9,1) eingesetzt ist,
wobei die obere Deckschichte (9) in der Zone des absorbierenden Körpers (2) der Langsrichtung des Produkts folgend in zwei oder mehr Falten (10) angeordnet ist, wobei die Bodenfelder (16), welche gegen den absorbierenden Körper (2) gerichtet sind, der Falten (10) am absorbierenden Körper (2) oder an einer Tragschichte (7), welche zwischen dem absorbierenden Körper (2) und der oberen Deckschichte (9) eingesetzt ist, fixiert sind und wobei die Bodenfelder (16) der Falten (10) am absorbierenden Körper (2) oder an der Stützschichte (7) über eine Breite von vorzugsweise 0,2 bis 10 mm geklebt oder geschweißt sind, dadurch gekennzeichnet, daß einige, vorzugsweise 2 bis 10, Falten (10) an einem begrenzten Seitenstreifen (18) der Oberfläche (6) des absorbierenden Körpers und/oder an einem Mittelstreifen (19) beigestellt sind.

2. Ein Hygieneprodukt nach Anspruch 1, dadurch gekennzeichnet, daß die Bodenfelder (16) zweier benachbarter Falten (10) mit einer gegenseitigen Beabstandung (A) von 1 bis 20 mm fixiert sind.

3. Ein Hygieneprodukt nach Anspruch 1 oder 2, dadurch gekennzeichnet, daß die Höhe (H) der Falten (10) in einem Zustand ohne Last 1 bis 20 mm beträgt.

## Revendications

1. Produit hygiénique absorbant pour l'absorption de liquides corporels. en particulier serviette hygiénique, couche, serviette pour l'incontinence, ou analogue, comprenant :
- une couche de recouvrement (1) imperméable aux liquides sur le fond,
- une couche de recouvrement (9) perméable aux liquides, de préférence réalisée avec un matériau non tissé, sur le dessus, et
- un corps absorbant (2) inséré entre les couches de recouvrement de fond et de dessus (9, 1).
dans lequel la couche de recouvrement de dessus (9) située sur la surface du corps absorbant (2) est agencée en deux plis (10), ou plus, suivant la direction longitudinale du produit, dans lequel des surfaces de pied (16), dirigées vers le corps absorbant (2) des plis (10), sont fixées au corps absorbant (2) ou a' une couche de support (7) insérée entre le corps absorbant (2) et la couche de recouvrement de dessus (9), et dans lequel les surfaces de pied (16) des plis (10) sont collées (17) ou réunies hermétiquement sur le corps absorbant (2) ou la couche de support (7) sur une largeur (F) comprise. de préférence, entre 0,2 et 10 mm, caractérisé en ce que plusieurs plis (10), de préférence 2 à 10, sont prévus sur une bande partielle latérale (18) de la surface (6) du corps absorbant et/ou une bande centrale (19).

2. Produit hygiénique selon la revendication 1, caractérisé en ce que les surfaces de pied (13) de deux plis adjacents (10) sont fixées avec un espacement mutuel (A) compris entre 1 et 20 mm.

3. Produit hygiénique selon la revendication 1 ou la revendication 2, caractérisé en ce que la hauteur (H) des plis (10) est comprise entre 1 et 20 mm dans un état non chargé.
